Europäisches Patentamt

European Patent Office

Office européen des brevets

(11) Publication number : **0 583 894 A2**

(12) # EUROPEAN PATENT APPLICATION

(21) Application number : 93305866.1

(22) Date of filing : 26.07.93

(51) Int. Cl.$^5$ : **G01N 33/68,** G01N 33/92, C12Q 1/68

The applicant has filed a statement in accordance with Rule 28 (4) EPC (issue of a sample only to an expert). Accession number(s) of the deposit(s): ATCC CRL 11088.

The applicant has subsequently filed a sequence listing and declared, that it includes no new matter.

(30) Priority : **29.07.92 US 922494**

(43) Date of publication of application :
**23.02.94 Bulletin 94/08**

(84) Designated Contracting States :
**AT BE CH DE DK ES FR GB GR IE IT LI LU NL PT SE**

(71) Applicant : **HYBRITECH INCORPORATED**
**11095 Torreyana Road**
**San Diego, California 92196-9006 (US)**

(72) Inventor : **David, Gary Samuel**
**9477 Poole Street**
**LaJolla, California 92037 (US)**
Inventor : **Hale, John Edward**
**9870 La Tortola Place**
**San Diego, California 92129 (US)**
Inventor : **Richard III, Charles**
**1098 Oceanic Drive**
**Encinitas, California 92024 (US)**
Inventor : **Nakamura, Kevin Kei**
**2409 Newcastle Avenue**
**Cardif, California 92007 (US)**

(74) Representative : **Hudson, Christopher Mark et al**
**Erl Wood Manor**
**Windlesham Surrey GU20 6PH (GB)**
**Declaration under Rule 28(4) EPC (expert solution)**

(54) **Method of immobilizing and orienting molecules by use of metal chelating moieties to facilitate atomic force microscopy of proteins and molecular scale manipulation.**

(57) An IgG$_1$ was engineered for specific adherence to a metal-containing surface by the incorporation C-terminal metal-chelating peptide (CP) and was bound to a nickel-coated mica surface. The metal-chelating peptide intereaction enabled the imaging of the proteinusing atomic force microscopy (AFM). The homogeneous appearance and calculated dimensions of the CP derivatized protein demonstrated the regiospecific orientation and stability imparted by this peptide. The ability to stably orient biomolecules to a surface may be used advantageously in a variety of processes designed to either visualize or manipulate molecular entities.

EP 0 583 894 A2

Atomic Force Microscopy (AFM) is a highly useful tool allowing visualization of three dimensional structures of molecular size, for example, proteins and polynucleotides. AFM and similar atomic scale visualization instruments, such as the Scanning Tunneling Microscope (STM) are serving as prototypes for the development of tools to not only visualize, but to manipulate individual molecules and atoms.

To realize the utility of these instruments, it is necessary to immobilize the molecules to be visualized or manipulated so that the mechanical forces involved in the procedure do not physically remove the molecules from the substrate on which they are located. To optimally realize the value of the instruments, it is also important in most cases to specifically orient the molecules for visualization of certain regions or to permit specific manipulation of portions of the molecules, for example, attachment of a second molecular species to a location on the anchored molecule.

Attachment of molecules to a substrate for observation or manipulation in the AFM has proven difficult. For example, Hansma et al. [(1992) Science 256:1180-1184] found it necessary to immerse plasmid DNA in propanol to prevent its movement in the AFM. DNA is insoluble in propanol, and under those conditions it was found to be minimally disturbed by the force of the AFM tip. Lin, et al [(1990) Langmuir 6:509) describe the imaging of an IgG molecule by AFM. However they indicate that they could not achieve adherence to the surface without the protein forming "lateral interactions" among molecules resulting in a random latticework of IgG molecules which remained bound to the surface. They were not able to achieve sufficient binding of individual protein molecules to the surface to allow AFM imaging.

It is undesirable to resort to denaturing conditions (such as a solvent in which the molecule cannot exist in its native state) to visualize or manipulate a molecule, because under those conditions the conformation of the molecule is unlikely to represent that found under native conditions. We describe here a means of anchoring the molecule for either observation or manipulation by tools which operate at the molecular level. In addition, the ability to control the location of the immobilization site provides an opportunity to orient the molecule. Immobilization can occur under native, non-denaturing conditions.

The instant invention provides a means to furnish a stable bond between a AFM Substrate and a molecule through the use of metal chelating agents and metallicized AFM Substrates. The instant invention also provides a means to regiospecifically anchor a molecule for purposes of further manipulation. The instant invention also provides a means to position molecules on a surface when those molecules are bound to the surface through a metal chelating moiety. The instant invention also provides molecularly - AFM Substrates where a molecule is bound to the surface through a transition metal ion complex.

One aspect of the instant invention provides a method to anchor proteins to an AFM Substrate to allow AFM imaging of the protein through the use of organic or peptidyl metal chelating agents. A wide variety of organic chelating agents are known in the art. Chelating peptides are amino acid sequences which are able to interact with and bind to metal ions. Smith, et al. (1988) J. Biol. Chem. 263, 7211-7215. They may be specifically designed, as described in the above reference, or can be naturally occurring within a protein. A protein possessing a chelating peptide (herein termed a CP-protein) or other organic chelating agent will bind to a metal ion immobilized on an AFM substrate with high specificity in a manner sufficiently stable to resist the disrupting forces encountered during interaction with a mechanical entity such as an AFM tip.

In the practice of the invention as exemplified herein, a CP-protein was prepared using recombinant PCR (R. Higuchi, in PCR Technology; Principles and Applications for DNA Amplification, H.A. Erlich, ed. (1989) Stockton Press, New York, NY) to obtain a gene coding for a gamma-1 heavy chain with a C-terminal chelating peptide. Immobilized metal affinity chromatography (CP-IMAC) was used to isolate and purify the CP-gamma-1 immunoglobulin (J. Porath, et al., (1983) Biochem. 22; 1621; E. Sulkowski, (1985) Trends in Biotech 3:1; M.C. Smith, et al., (1987) Inorg. Chem 26:1965; E. Hochuli, et al., (1987) J. Chrom. 411:177; M.C. Smith, et al, (1988) J. Biol. Chem. 263:7211; F. H. Arnold (1991) Bio/Technology 9:151). Atomic force microscopy was carried out to illustrate and visualize the stability and surface orientation of the engineered antibody. The images of the immobilized antibody shown in the accompanying drawings demonstrate the significant advances in both the atomic force microscopy of proteins and the regiospecific orientation and stabilization of a biomolecule to a tangential surface provided by the instant invention. The ability to orient and position proteins and other molecules immobilized on an AFM Substrate according to the practice of the present invention facilitates the molecular manipulations necessary for realization of the practical utility of an environment on a molecular scale.

Furthermore, the instant invention also provides methods for the visualization of stable protein/protein or protein/substrate interactions. Complexes may be immobilized via a metal chelating moiety to allow visualization of not only the protein but how the protein interacts with its substrate/ligand/receptor. This is particularly useful for the study of enzyme action, antibody-antigen recognition, etc. This information will be useful for the structure based design of receptor/ligand systems for in vitro or in vivo use.

AFM Substrate -- An atomically flat and smooth surface suitable for high resolution microscopy, including but not limited to natural or synthetic polymers such as mica, graphite, gallium arsenite, glass, plastics, metal, cyanoacrylates, iron, and the like.

Analog -- a compound which is functionally similar to another yet possesses a different structure.

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with U or C with G.

Molecular Entity (ME) -- refers to compounds chosen from the group consisting of IPs, proteins, nucleotides, polynucleotides, lipids, carbohydrates, enzymes, drugs, organic, organometallic or inorganic molecules or polymers and cofactors.

CHEL 13 -- the XCEM 449 antibody (Beidler, et al. (1988) J. Immunology 141:453-460 herein incorporated by reference) modified to incorporate a chelating peptide at the carboxy terminus of the heavy chain.

Chelating Peptide (CP) -- an amino acid sequence capable of complexing with a metal ion having multiple coordination sites. The presence of the CP designation when associated with another molecule, such as "CP-enzyme", indicates that the molecule is covalently linked to a chelating peptide.

Chelator -- an organic chelating agent or chelating peptide.

Chelating Peptide Immobilized Metal Ion Affinity Chromatography (CP-IMAC) -- a method of linking a fusion protein containing a chelating peptide or a free chelating peptide to a solid support where this solid support possesses a coordinately complexed metal ion as described in United States Patent 4,569,794, the entire teaching of which is hereby incorporated by reference.

CP-protein -- a protein comprising an amino acid sequence of a protein directly or indirectly covalently bound to a chelating peptide.

CP-molecule -- a hybrid molecule comprising the amino acid sequence of a chelating peptide directly or indirectly covalently bound to a molecule.

Derivatized AFM Substrate - an AFM Substrate possessing a bound metal ion to which a molecule has been bound through the use of chelating function on the molecule.

Functional analog -- refers to a molecule having similar functional properties but a modified structure relative to the naturally occurring form of that molecule or compound. A functional analog includes fragments of or additions to the parent molecule which have similar functional properties and reactivities as the parent molecule.

Fusion Protein -- refers to a polypeptide which comprises the amalgamation of two amino acid sequences derived from heterogeneous sources.

IDA -- an abbreviation for iminodiacetic acid.

IMAC -- an abbreviation for immobilized metal ion affinity chromatography.

Immunoreactive Protein(s) (IP)-- a term used to collectively describe antibodies, fragments of antibodies (such as, but not limited to, Fab, Fab', Fab2',and Fv fragments), and chimeric, humanized, veneered, or CDR-grafted antibodies or antibody derivatives capable of binding antigens of a similar nature as the parent antibody molecule from which they are derived, and "Single Chain Polypeptide Binding Molecules". IP includes MAB 35 as described by Jean Pierre Mach and associates, CHA 255, CEM231, and XCEM449 (Beidler, et al.).

Milli-Q® water -- water purified through the Milli-Q® reagent water system available from Millipore Corporation, Ashby Road, Bedford, MA 01730.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- a recombinant DNA cloning vector in which a particular DNA sequences facilitating the expression of heterologous proteins have been incorporated.

Replicon -- a DNA sequence that controls and allows for autonomous replication of a plasmid or other vector.

Reporter molecule (or reporter group) -- refers to compounds commonly used in diagnostic applications such as visual dyes, fluorescent chelating agents, radioactive compounds, and other compounds which possess photo-metrically useful properties

Single Polypeptide Chain Binding Molecule -- a polypeptide possessing immunological specificity as described in PCT Application No. PCT/US 87/02208, International Publication No. WO 88/01649, International Publication Date: 10 March 1988, the entire teaching of which is hereby incorporated by reference.

Transition metal ion complex -- for purposes of the present invention, a transition metal ion complex refers to the interaction between a molecule possessing a chelating moiety and a transition metal ion, wherein said metal ion is bound to the AFM Substrate and said chelating moiety.

Vector -- a replicon used for gene manipulation bearing polynucleotide sequences corresponding to appropriate protein molecules which, when combined with appropriate control sequences, confers specific properties on the host cell to be transformed. Plasmids, viruses, and bacteriophages are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors. When vectors are introduced to bacterial cells the process is termed transformation. The analogous process in eucaryotic cells is termed transfection.

The restriction site and function maps presented in the accompanying drawings are approximate representations of the recombinant DNA vectors discussed herein. The restriction site information is not exhaustive; therefore there may be more restriction sites of a given type on the vector than are illustrated in the drawings.

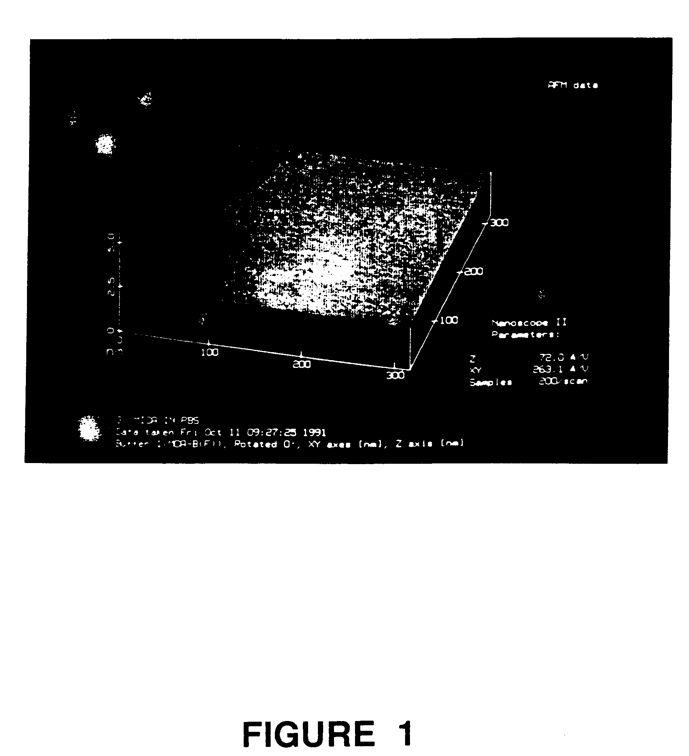

Figure 1 -- Atomic force microscopy image of a freshly cleaved mica surface imaged under PBS, pH 7 using a 12 micrometer scanner head and a 100 micrometer cantilever. The force is approximately $10^{-7}$ newtons.

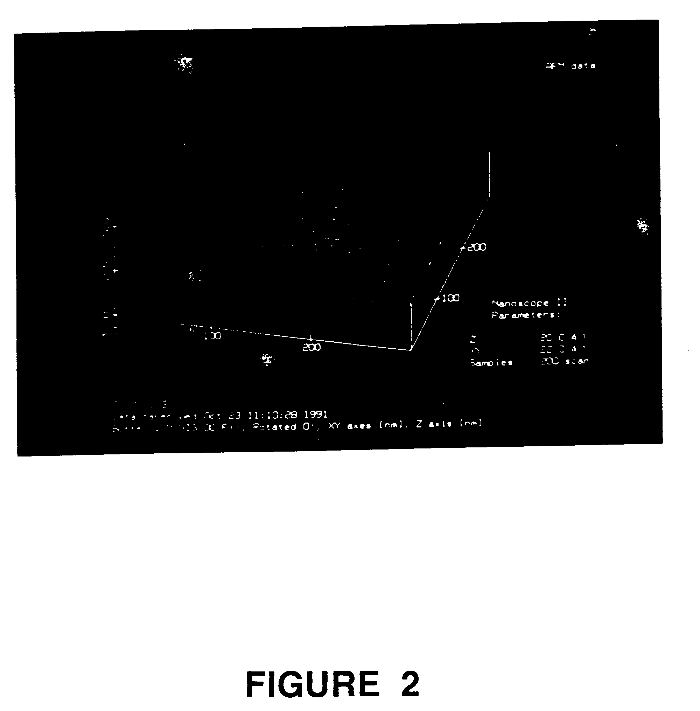

Figure 2 -- Atomic force microscopy image of a nickel impregnated mica surface. 5 $\mu$l of 1 mM nickel chloride was spotted on a freshly cleaved mica surface, allowed to sit for 1 minute, and rinsed with Milli-Q® water. This was air-dried at room temperature for 20 minutes, and imaged under PBS, pH 7 using a 12 micrometer scanner head and a 100 micrometer cantilever. The force is approximately $10^{-7}$ newtons.

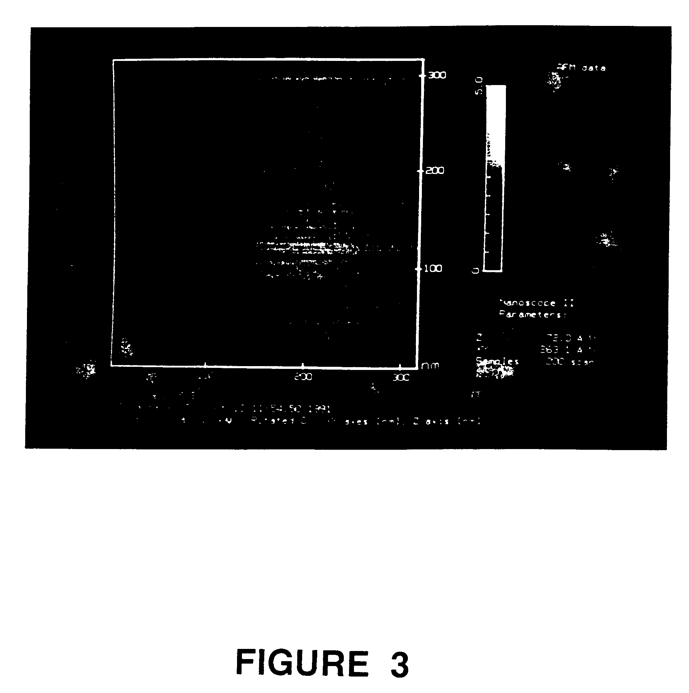

Figure 3 -- Atomic force microscopy image of a Mica-CHEL 13. 100 $\mu$l of 0.5 $\mu$g/ml CHEL 13 was injected into the fluid cell on a freshly cleaved mica surface, and allowed to be in contact with the mica surface for 10 minutes. The fluid cell was then flushed with PBS, and imaged under PBS, pH 7 using a 12 micrometer scanner head and a 100 micrometer cantilever. The force was approximately $10^{-9}$ Newtons.

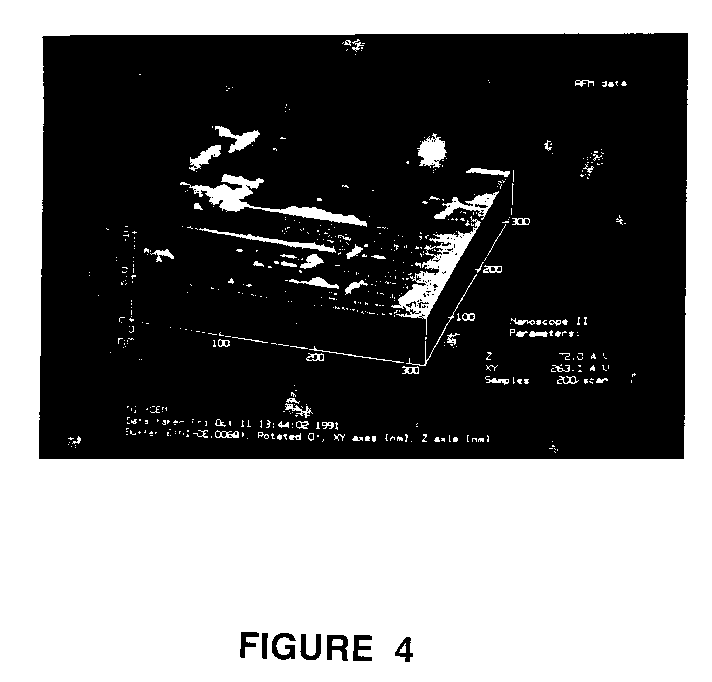

Figure 4 -- Atomic force microscopy image of a Mica- Nickel with XCEM 449. 5 $\mu$l of 1 mM Nickel chloride was spotted on a freshly cleaved mica surface, allowed to sit for 1 minute, and rinsed with Milli-Q® water. This was air-dried at room temperature for 20 minutes, and attached to a fluid cell. 100 $\mu$l of 0.5 $\mu$g/ml of XCEM 449 antibody was injected into the fluid cell for 10 minutes and then flushed with PBS. Images were made under PBS using the 12 micrometer scanner head and a 100 micrometer cantilever. The force was approximately $10^{-9}$-$10^{-10}$ Newtons.

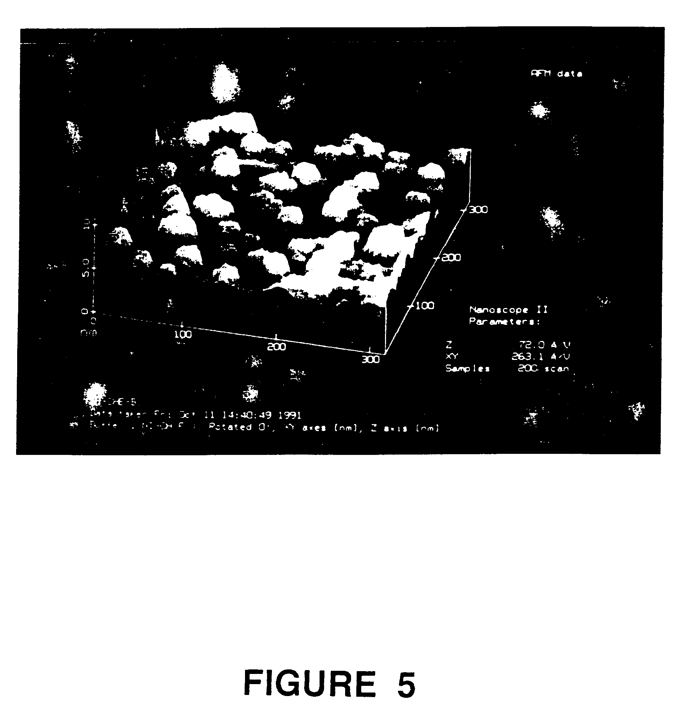

Figure 5 -- Atomic force microscopy image of a nickel impregnated mica surface exposed to CHEL 13. 5 $\mu$l of 1 mM nickel chloride spotted on a freshly cleaved mica surface, allowed to sit for 1 minute, and rinsed with Milli-Q® water. This was air-dried at room temperature for 20 minutes and attached to the fluid cell. 100 $\mu$l of 0.5 $\mu$g/ml of CHEL 13 was injected into the fluid cell and allowed to be in contact with the nickel impregnated mica surface for 10 minutes. The fluid cell was then flushed with PBS and imaged under PBS using a 12 micrometer scanner head and a 100 micrometer cantilever. The force was approximately $10^{-9}$-$10^{-10}$ Newtons.

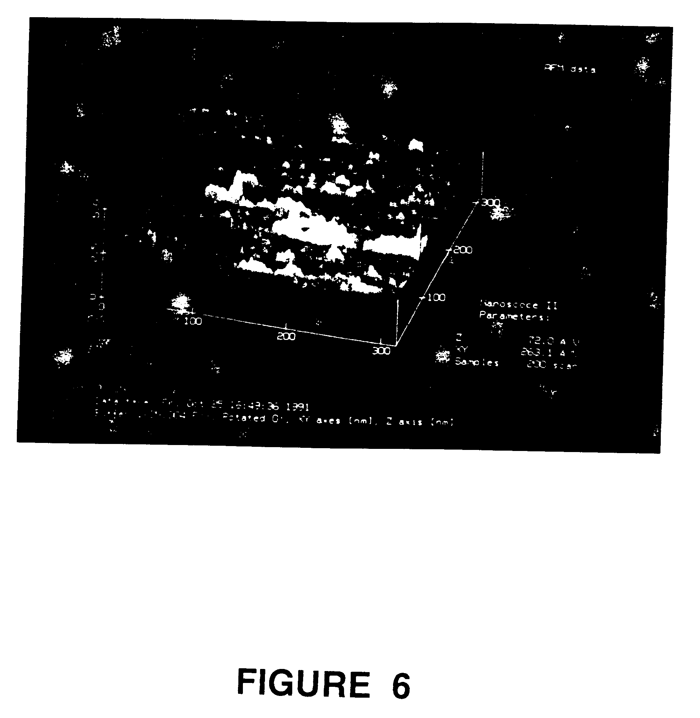

Figure 6 -- Atomic force microscopy image of the nickel impregnated mica surface exposed to CHEL 13 containing bound nickel. 5 $\mu$l of 1 mM nickel chloride was spotted on a freshly cleaved mica surface, allowed to sit for 1 minute, and rinsed with Milli-Q® water. This is air dried at room temperature for 20 minutes, and attached to the fluid cell. 180 $\mu$l of CHEL 13(0.5 $\mu$g/ml) was pre-incubated for 30 minutes with 20 $\mu$l of nickel chloride (10 mM). 100 $\mu$l of this material was injected into the fluid cell and incubated for 10 minutes with the Nickel-mica surface. The fluid cell was then flushed with PBS and imaged under PBS, using a 12 micrometer scanner head and a 100 micrometer cantilever. The force was approximately $10^{-9}$-$10^{-10}$ Newtons.

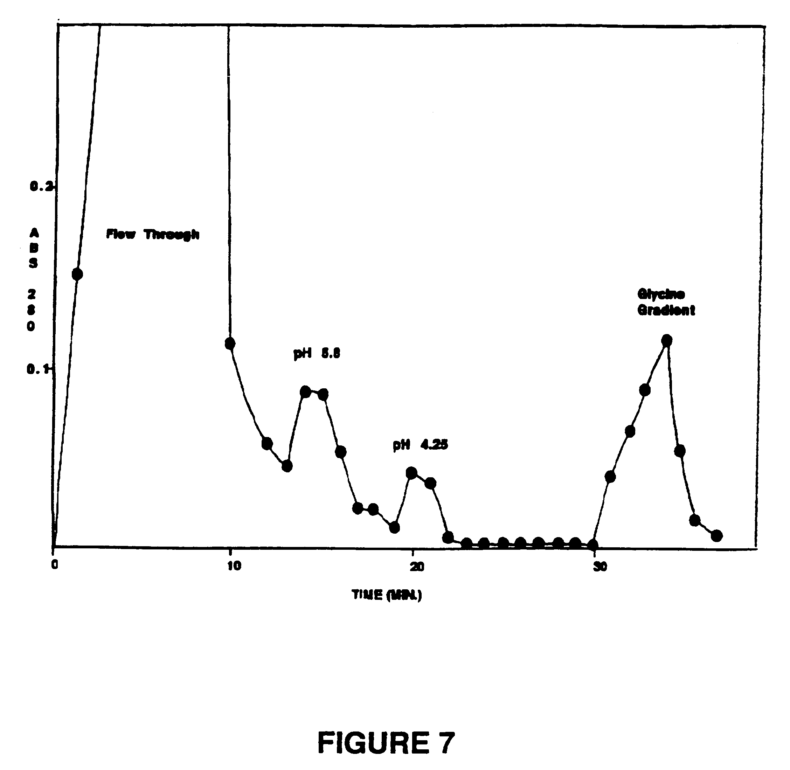

Figure 7 -- A chromatogram of nickel-loaded IDA HPLC column indicating the separation of antibody and fragments. The column eluent yielded the 4-peaks indicated in the Figure the presence of protein being determined by $A_{280}$.

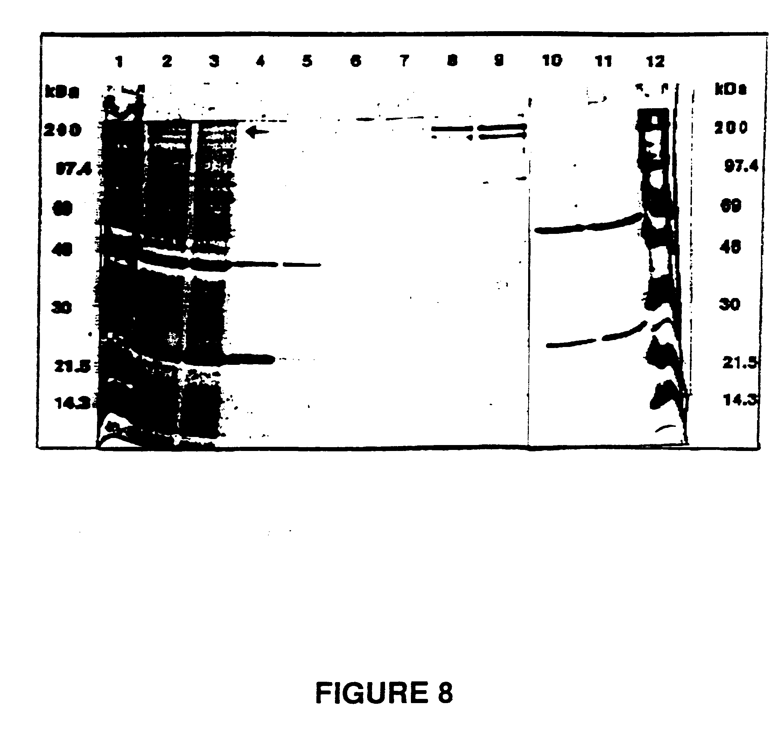

Figure 8 -- A 12% SDS-PAGE gel analyzing the peaks from the nickel-loaded IDA HPLC column visualized by staining with Comassie blue.

The instant invention provides a complex of the formula:

ME-(spacer)$_x$-Chelator-(M)-AFM Substrate          (I)

wherein:

"ME" is an Molecular Entity,

x = 0 or 1,

"M" is a transition metal ion,

with the proviso that multiple [ME-(spacer)$_x$-Chelator-(M)-] units may be bound to the single AFM Substrate and that each [ME-(spacer)$_x$-Chelator-(M)-] unit may be the same or different.

The instant invention further provides a method of making the complex of formula I said method comprising the steps of:

a.) synthesizing a [ME-(spacer)$_x$-Chelator-] unit

wherein:

"ME" is an Molecular Entity,

x = 0 or 1, and

b.) reacting the ME-(spacer)$_x$-Chelator- unit with an AFM Substrate possessing a bound transition metal ion.

The instant invention further provides a method of making the complex of formula I, said method comprising the steps of:

a.) synthesizing a [ME-(spacer)$_x$-Chelator-(M)] unit

wherein:

"ME" is an Molecular Entity,

x = 0 or 1,

"M" is a transition metal ion, and

b.) reacting the ME-(spacer)$_x$-Chelator-(M) unit with an AFM Substrate substantially free of bound metal ions.

The instant invention further provides a method of manipulating a Molecular Entity (ME) immobilized on an AFM Substrate using the tip of a scanning probe microscope.

The instant invention further provides complexes synthesized by this method.

Molecular Entities (MEs) incorporated into the complexes of formula I of the present invention are selected from the group comprising proteins, enzymes, drugs, nucleotides, IPs, carbohydrates, lipids, or organic, organometallic, inorganic molecules, protein-substrate complexes, protein-ligand complexes, or polymers. In preferred compounds of the invention, said Molecular Entity (ME) is protein. In further preferred compounds of the invention, said Molecular Entity is an IP. In further preferred compounds of the invention, said ME is an antibody. In further preferred compounds of the invention, said IP is an Fab, Fab' or Fv molecule. In further preferred compounds of the invention, said Molecular Entity is a chimeric, humanized, veneered, or CDR-grafted antibody. In further preferred compounds of the invention, said ME is the HIV protease. In further preferred compounds of the invention, said ME is the RB protein.

In some instances, such as where steric hindrance may interfere with the formation of the metal ion coordination complex between the AFM Substrate and the chelating moiety of the ME, it may be desirable to provide a "spacer" between the chelating moiety and the ME sought to be immobilized. For example, in designing an CP-protein, it is desirable to prevent disturbances to the functional and structural portions of the native protein molecule. Molecular modeling systems such as those available from Silicon Graphics (Palo Alto, CA) [using Insight II software from Biosym (San Diego, CA)] may be used to assist in the design of a CP-protein or other Chelator derivatized ME so as to minimize the steric interference between the chelating agent and the ME.

In the practice of the present invention a "spacer" denotes a polypeptide, protein, polysaccharide, polynucleotide, or a synthetic organic moiety composed of from 1 to 50 monomer units. It is preferred that from 5 to 50 monomer units (e.g., amino acids, hexoses, pentoses, nucleotides, methylene groups, methenyl groups, acrylic acid residues, etc.) be used in the linker molecule. Chelators need not be at the ends of the linker molecule. Where the ME is a CP-protein, a peptidyl spacer of from 1 to approximately 30 amino acids may be used where steric hindrance between the protein and the chelating peptide is believed to be a potential problem. The inclusion of a polypeptide spacer is readily achieved when the CP-protein is produced via recombinant DNA technology by inserting a DNA sequence encoding the desired polypeptide spacer (preserving the proper reading frame of the chelating peptide and protein molecules) between the DNA sequences encoding the chelating peptide and the protein sought to be immobilized. Chelating peptides may also be linked to proteins through organic or organometallic linkers of varying length to provide the desired spacing effect.

The term "Chelator" refers to a transition metal ion chelating moiety capable of binding to one or more coordination sites of a transition metal ion to achieve a coordinate covalent complex between the protein and the metal ion. The molecule may have the metal ion bound through the use of a chelating peptide or other multidentate chelating moiety. The Chelator may be coupled to the ME by chemical means well known in the art. For example, see Means, G.E. and Feeney, R.E., Chemical Modifications of Proteins, Holden Day, San Francisco, 1971.

In one embodiment of the present invention, chelating peptides (CPs) may be incorporated into the primary structure of a protein as a Chelator. Such proteins are termed CP-proteins. The use of CP-protein immobilization was first described in reference to the CP-IMAC purification technique. The principles and practice of the CP-IMAC purification procedure are provided in Smith, M.C. and Pidgeon, C. United States Patent No. 4,569,794, issued February 11, 1986, the entire teaching of which is hereby incorporated by reference. Virtually any protein may be incorporated into a CP-protein form. The chelating peptide domain of the CP-protein will bind to an immobilized metal ion.

Chelating peptides employed in the practice of the present invention are represented by the formula:

$$(His)_x-(A)_y-(His)_z$$

where A is an amino acid,

$x=0-10,$

$y=0-4,$

$z=0-10,$

and polymers thereof wherein each monomer unit of said polymer is the same or different.

In the preferred practice of the invention, the chelating peptide may be from 2 to 10 amino acids in length containing from at least one, and preferably 2 or more, histidine residues. In the preferred practice of the invention A is selected from the group comprising Trp, Gly, or Tyr. Those chelating peptides most preferred in the practice of the instant invention are shown in Table I below:

---

### Table I. Chelating Peptides

| letter | amino acid sequence | Seq. ID# |
|---|---|---|
| a) | His-Trp-His-Met-Tyr | (Seq.ID#1) |
| b) | His-His-His-Met-Tyr | (Seq.ID#2) |
| c) | His-His-His-His-Tyr | (Seq.ID#3) |
| d) | His-Trp-His-Trp-His | (Seq.ID#4) |
| e) | His-Trp-His-His-His | (Seq.ID#5) |
| f) | His-His-His-His-Tyr-Met-His-His-His-His-Tyr | (Seq.ID#6) |
| g) | His-His-His-His-His | (Seq.ID#7) |
| h) | His-Trp-His-His-His-Pro | (Seq.ID#8) |
| i) | His-Gly-His-Gly-Gly-Gly-His-Gly-His | (Seq.ID#9) |
| j) | His-Gly-His-Gly-Gly-Gly-Gly-Gly-Gly-His-Gly-His | (Seq.ID#10) |

---

It is permissible to have an N-terminal methionine residue, characteristic of recombinant proteins, and still retain the functional aspects of the chelating peptide sequences shown above when the CP is at the amino terminus of the recombinant CP-protein. Furthermore, it is permissible to have from 1 to approximately 12 amino acids between the N-terminus of the CP-protein and the chelating peptide and retain the chelating properties of the chelating peptide. The determination of how many amino acids may be placed between the chelating peptide and the structural protein is dependent on the properties of the structural protein. Accordingly, the peptide spacer should be chosen so as to not interfere with the physical characteristics of the structural protein. It is also permissible to have from 1 to approximately 12 amino acids following the chelating peptide when the chelating peptide is located at the C-terminus of the protein. It is also permissible to incorporate the chelating peptide into the primary structure of the protein so that it is surface exposed and capable of complexing with a metal ion.

CP-proteins may be formed by a variety of methods. CP-proteins may be produced either by recombinant DNA technology or well known chemical procedures, such as solution or solid-phase peptide synthesis, or semi-synthesis in solution beginning with protein fragments coupled through conventional solution methods.

The principles of solid phase chemical synthesis of polypeptides are well known in the art and may be found in general texts in the area such as Dugas, H. and Penney, C., Bioorganic Chemistry (1981) Springer-Verlag, New York, pgs. 54-92. For instance, the CP-protein may be synthesized on an Applied Biosystems Model 431 peptide synthesizer (commercially available from Applied Biosystems Inc., Foster City, CA, USA) in substantial accordance with the directions supplied by the manufacturer.

In the preferred practice of the invention, CP-proteins are produced through the use of recombinant DNA technology by the addition of an in-frame DNA sequence encoding a chelating peptide into the coding sequence of the protein of interest. It is well known in the art how to construct a DNA sequence encoding a given amino acid sequence. Brown, et al. Methods in Enzymology 68:109 (1979). To effect the translation of the desired polypeptide, one inserts the engineered synthetic DNA sequence encoding the CP-protein in any of a plethora of appropriate recombinant DNA expression vectors through the use of appropriate restriction endonucleases. The particular endonucleases employed will be dictated by the restriction endonuclease cleavage pattern of the parent expression vector to be employed. The CP-protein coding sequence is positioned so as to be in proper reading frame with the promoter and ribosome binding site of the expression vector, both of which are functional in the host cell in which the CP-protein is to be expressed. A preferred vector for expression in an E. coli host cell is derived from E. coli plasmid pHS190, which comprises the TcR gene, the lambda cI857 repressor and the lambda pL promoter. Plasmid pHS190 is on deposit with the Northern Regional Research Laboratories under the accession number NRRL B-18410 (date of deposit: September 9, 1988).

Incorporation of the chelating peptide coding sequence may be achieved by conventional genetic cloning techniques described above or through the use of the Polymerase Chain Reaction (PCR) to create and amplify the synthetic gene encoding the CP-protein. A description of the principles and techniques involved in the use of PCR is provided in Molecular Cloning: A Laboratory Manual, 2nd. Edition, Sambrook, A, Fritsch, E.F., and Maniatis, T. (1989) Cold Spring Harbor Press. A description of the principles and techniques involved in the use of recombinant PCR (R. Higuchi, in PCR Technology: Principles and Applications for DNA Amplification, H.A. Erlich, ed. Stockton Press, New York, NY (1989). Primers for use in the PCR process may be synthesized by techniques well known in the art such as by employing an Applied Biosystems Model 380A or 380B DNA synthesizer (commercially available from Applied Biosystems, Foster City, California) in substantial accordance with the directions provided by the manufacturer. Synthetic PCR primers may be created which contain the coding sequence of the chelating peptide thereby directly incorporating the chelating peptide coding sequence into the DNA produced via PCR. The expression products of such coding sequences are "CP-proteins" which possess a chelating peptide.

The placement of the chelating peptide in the ultimate CP-protein is largely dictated by the properties of the protein. Preferably, the chelating peptide coding sequence may be incorporated into the protein coding sequence at or near either the 5' or 3' ends of the protein coding sequence such that the chelating peptide will occur at or near either the amino or carboxyl terminus of the protein. Furthermore, the chelating peptide may be incorporated into the primary structure of a protein so that it is surface exposed and is capable of complexing with a metal ion. In all circumstances, it is preferred that the chelating peptide should be incorporated into the protein so as to minimize any potentially negative effect on the desirable properties of the protein. In designing an CP-protein, it is desirable to prevent disturbances to the functional and structural portions of the native protein molecule. Molecular modeling systems such as those available from Silicon Graphics (Palo Alto, CA) using Insight II software from Biosym (San Diego, CA)) may be used to assist in the design of a CP-protein so as to minimize the interference between the chelating agent and the functional domains of the protein.

Immunoreactive Proteins may also be modified by derivatization with a Chelator allowing such derivatized IPs to be used in the practice of the instant invention. In the preferred practice of the invention as exemplified herein, IPs are incorporated into CP-protein form. As previously described, a CP-IP may be synthesized by recombinant DNA technology or well known chemical procedures, such as solution or solid-phase peptide synthesis, or semi-synthesis in solution beginning with protein fragments coupled through conventional solution methods. Recombinant CP-IPs (CP-IPs) are produced by inserting a DNA sequence encoding a chelating peptide in the region of the coding sequence distal to the DNA sequence encoding the antigen binding site of the molecule. Such CP-IPs possess the immobilized metal ion binding capabilities of other CP-proteins. Where one is using the method of this invention to maximize the exposure of the antigen binding site of such CP-IPs, it is preferred to attach the chelating moiety distal to the antigenic binding site of IP molecule. These CP-IPs may then be immobilized on an AFM Substrate through a coordinate covalent complex involving a transition metal ion.

The DNA coding and amino acid sequences of a wide variety of IP molecules are known. Such IPs could be converted to CP-IP form and produced by synthetic, semi-synthetic or recombinant means. The DNA coding sequence encoding a variety of IP molecules have been isolated facilitating recombinant production of such CP-IPs. Examples of such IPs include but are not limited to, fragments of antibodies (such as but not limited to Fab, Fab', Fab2', and Fv fragments), and chimeric, humanized, veneered, or CDR-grafted antibodies capable of binding antigens and "single chain polypeptide binding molecules". Preferred IPs include a CEM231, a CEL 007, or Mab 35 antibody or an antigen binding fragment thereof, a CHA 255 antibody or a fragment thereof, and the Fv fragment of CEM231.6.7.

The Chelator may also be an organic chelating agent selected from the group comprising bidentate, tridentate, quadridentate, tripod, and macrocyclic ligands. Examples of such organic chelating agents include bidentate, tridentate, and quadridentate ligands such as iminodiacetic acid (IDA), nitrilotriacetic acid (NTA), terpyridine, phenanthroline, bipyridine, triethylenetetraamine, biethylenetriamine and tripod ligands such as the polypyrazolyl borate ligands, and macrocyclic ligands such as 1,4,7 triazacyclononane, dimethylglyoxime, diphenylglyoxime, and the like.

"M" is a transition metal ion capable of forming a kinetically labile or inert transition metal ion complex. A variety of metal ions may be incorporated into the practice of the instant invention. Those of ordinary skill in the art will appreciate the parameters involved in incorporating these metal ions into the practice of the instant invention.

Certain metal ions are preferred for use with particular chelating agents. In general, for the purposes of this invention, "hard" metal ions should be complexed with harder ligands and "soft" metal ions should be complexed with soft ligands. Pearson, R.G. (1966) Science 151:172-177. For example, when the chelating agent employed is a harder ligand such as iminodiacetic acid (IDA) or nitrilotriacetic acid (NTA), preferred metal ions include Mn(II/III), V(II/III/IV), Cr(II/III), Fe(II/III), Co(II/III), Ni(II), Cu(II/III), Zn(II), In(III), La(III), Gd(III), Lu(III), Y(III), Pr(III), Nd(III), Sm(III), Eu(III), Tb(III), Dy(III), Ho(III), Er(III), Tm(III), Yb(III), Al(III), Sc(III), Ga(III), Ce(III/IV), Zr(IV), Th(IV), Hf(IV), Tl(III), Si(IV), Ti(IV), Sn(II/IV), and Os(II). When the chelating agent employed in the practice of the present invention is a harder ligand, the most preferred metal ions include Mn(II/III), V(II/III/IV), Cr(II/III), Fe(II/III), Co(II/III), Ni(II), Cu(II/III), and Zn(II).

When the chelating agents employed is a soft ligand such as triethylenetetraamine, biethylenetetraamine, or 1,4,7-triazacyclonane, Fe(II), Co(II/III), Ni(II), Cu(I/II), Pt(II/IV), Pd(II), Cd(II), Au(III), Rh(III), Ir(III), Mo(V/VI), Os(II/III), and Zn(II), Mn(II), Fe(II/III), Co(II), Ni(II), Cu(I/II), zn(II), Cd(II), Ga(III), In(III), Tl(III). Robert M. Smith & Arthur E. Martell, Critical Stability Constants, Plenum Press, New York, Vol 1 (1974), Vol 2 (1975), Vol 5 (1982), and Vol 6 (1989).

When the chelating agents employed is of intermediate hardness such as terpyridine, bipyridine, or polypyrazolyl borate ligands, preferred metal ions include Mn(II/III), V(II/III/IV), Cr(II/III), Fe(II/III), Co(II/III), Ni(II), Cu(II/III), Zn(II), In(III), La(III), Gd(III), Lu(III), Y(III), Pr(III), Nd(III), Sm(III), Eu(III), Tb(III), Dy(III), Ho(III), Er(III), Tm(III), Yb(III), Al(III), Sc(III), Ga(III), Ce(III/IV), Zr(IV), Th(IV), Hf(IV), Tl(III), Si(IV), Ti(IV), Sn(II/IV), Os(II/III), Cu(I/II), Pt(II/IV), Pd(II), Cd(II), Au(III), Rh(III), Ir(III), and Mo(V/VI).

The [M-Chelator-ME] complex is immobilized on an AFM Substrate. Useful AFM Substrates include any atomically flat and smooth surface suitable for high resolution microscopy. Natural or synthetic polymers substrates such as mica, graphite, gallium arsenite, glass, plastics, metal, cyanoacrylates, iron, and the like are useful in the practice of the instant invention. Some AFM Substrates, such as the silicates (e.g., mica, glass, etc.) inherently possess the ability to bind the transition metal ion through the lone pairs of electrons present on the oxygen atoms of the octahedral silicate surface. Other AFM substrates lacking this capacity (such as polystyrene, polyethylene, etc.) may be derivatized with a Chelator using conventional chemical techniques.

Formation of the [AFM Substrate-M-Chelator-ME] complex may proceed in a variety of manners. One may initially create a complex between the Chelator-ME and the metal ion and then expose this [M-Chelator-ME] complex to the AFM Substrate or, as exemplified herein, form the [M-AFM Substrate] complex and then expose the Chelator-ME to the [M-AFM Substrate]. A variety of other iterations of the above scenarios are readily apparent and are within the scope of the instant invention.

Furthermore, the AFM Substrate could be chemically derivatized with a chelating agent to hold the Metal ion in place rather than rely on the natural metal binding properties of the AFM Substrate itself. For example, a plastic surface could be derivatized to possess an organic chelating agent as described above. This organic chelating agent could then be saturated with a metal or provide the basis for a [ME-Chelator-M] complex to bind to the derivatized AFM Substrate surface.

Furthermore, where steric hindrance between the CP-protein or CP-molecule and a metal derivatized AFM Substrate is required, rather than re-engineer the Chelator-ME to incorporate a spacer, a multiple chelating agent may be incorporated to position a second metal ion away from the AFM Substrate surface in a structure such as:

CP-protein - (M) --- polychelator --- (M) - AFM Substrate This "polychelator" may be a polypeptide, protein, polysaccharide, polynucleotide or a synthetic organic moiety composed of from 1 to 50 monomer units designed to possess a plurality of chelating functions. Again, the chelating functions of the polychelator need not be at the ends of the molecule.

In the practice of the method of the instant invention, the metal ion may be in a kinetically labile or inert oxidation state. Regarding the "kinetically inert complex" formed in the method, a distinction must be made between the terms "inert" and "stable". The term "inert" refers to the degree of lability. Lability refers to the ability of a particular complexed ion to engage in reactions that result in replacing one or more ligands in its coordination sphere by others. In an aqueous environment, the open coordination positions of the AFM Substrate-immobilized metal ion are occupied by water molecules. These water molecules must be displaced by the Chelator in order to form the [AFM Substrate-M-Chelator-ME] complex. When such reactions (ligand exchanges) occur rapidly, the reaction is termed "labile". However, where such reactions occur very slowly or not at all, the complex is said to be kinetically "inert".

Kinetic lability or inertness is related to the reaction rate and is not to be confused with thermodynamic stability or instability. Stability refers to the thermodynamic tendency of a species to exist under equilibrium conditions. Cotton and Wilkinson illustrate the point stating:

A simple example of this *[labile vs. stable]* distinction is provided by the $[Co(NH_3)_6]^{3+}$ ion which will persist for days in an acid medium because of its kinetic inertness or lack of lability despite the fact that it is thermodynamically unstable, as the following equilibrium constant shows:

$$[Co(NH_3)_6]^{3+} + 6H_3O^+ = [Co(H_2O)_6]^{3+} + 6NH_4^+ \quad K = 10^{25}$$

In contrast, the stability of $[Ni(CN)_4]^{2-}$ is extremely high:

$$[Ni(CN)_4]^{2-} = Ni^{2+} + 4CN^- \qquad K = 10^{-22}$$

but the rate of exchange of $CN^-$ ions with isotopically labeled cyanide ion added to the solution is immeasurably fast by ordinary techniques. Advanced Inorganic Chemistry, Cotton, F.A. and Wilkinson, G. (1972) 3rd. ed. Interscience Publishers, p.652.

Thus, the formation of the kinetically inert [ME-Chelator-M-AFM Substrate] complex assures the effective linkage of the ME to the AFM Substrate for periods of time ranging from days to years.

When forming a kinetically inert complex, there is a substantial advantage to forming the complex between the molecules containing the chelating moieties while the metal ion is in a labile oxidation state rather than simply using the metal ion in its kinetically inert oxidation state initially. The direct formation of a complex between the metal ion in its inert oxidation state and the molecules containing a chelating peptide would be impracticably slow.

As previously stated, the metal ion may be in a kinetically labile or kinetically inert oxidation state. When forming a kinetically inert complex, metal ions with octahedral complexes with half-filled ($d^3$) metal ions such as Cr(III), V(II), Mn(IV) and filled ($d^6$) low spin metal ions such as Co(III), Fe(II), Ru(II), Os(II), Rh(III), Ir(III), Pd(IV) and Pt(IV) tend to be extremely inert and useful in the practice of the instant invention. Hanzik, Robert P. in Inorganic Aspects of Biological and Organic Chemistry, Academic Press, New York, 1976, P. 109. See also Cotton, F. A. and Wilkinson, G. supra. In the most preferred practice of the invention it is desirable to proceed from Co(II) to Co(III), Cr(II) to Cr(III), or Ru(III) to Ru(II) to form the inert complex. The metal ions Zn, Cu, or Ni are useful in the practice of the present invention, but do not facilitate the formation of complexes as inert as those provided with the preferred metal ions, such as cobaltic ion.

Producing the necessary change in the oxidation state of the metal ion may be achieved by a variety of redox reagents. For example oxidizing agents such as oxygen, hydrogen peroxide, and peracids may be used in the practice of the invention. Examples of reducing agents include thiols, potassium ferrocyanide, potassium thiocyanate, sulfites, and sodium dithionite. These will be prepared in aqueous solutions of appropriate concentrations.

It will be recognized by those skilled in the art that not all redox reagents will be appropriate to change the oxidation state of these metal ions under all conditions so as to form kinetically inert complexes with the chelating agents provided. One factor in selecting the appropriate redox reagent is to take into consideration the electrochemical potential of the labile complex and use a reagent such that the overall free energy is such that reaction is spontaneous. It will also be recognized by those skilled in the art that the appropriate redox reagent may depend upon the nature of the ME function of the [ME-Chelator-M-AFM Substrate] complex. The susceptibility of proteins or reporter molecules to inactivation by certain redox reagents must be considered as well.

For example, a CP-protein may be immobilized to the AFM Substrate through a kinetically inert linkage utilizing cobaltic ion by the following procedure. The kinetically labile [ME-Chelator-Co(II)-AFM Substrate] complex is formed. The [ME-Chelator-Co(II)-AFM Substrate] complex is then exposed for 24 hours to an oxygen containing gas to oxidize the Co(II) to Co(III). The [ME-Chelator-M-AFM Substrate] complex thereby assumes the exchange inert conformation.

The ability to convert an inert bond to a labile bond is apparent in considering necessary atomic scale, single-molecule manipulations to carry out processes anticipated in the field of nanotechnology. One will be able to produce an inert linkage by a process such as that described above to firmly anchor a ME to the AFM surface for manipulations by means of a devise such as (but not limited to) an AFM tip. After manipulating the molecule one may change the oxidation state of the metal ion to recreate the kinetically labile conformation by the addition of the appropriate redox reagent. This will permit removal of the manipulated molecule so that it can be used for other purposes, should that purpose require the liberated molecule.

In designing an $IgG_1$ with an added chelating sequence the structural consequences were examined via Molecular Modeling to pre-examine the IgG1 for potential disturbances to the functional and structural portions of the native molecule. Inspection of the human $IgG_1$ crystal structure (J. Deisenhofer (1981) Biochemistry 20:2361 and use of the molecular modeling system from Silicon Graphics (Palo Alto, CA) using Insight II software from Biosym (San Diego, CA)) revealed an approximate 21 angstrom distance between the C-terminal lysines of the two heavy chains. The addition of the His-Trp-His-His-His-Pro sequence was thus hypothesized not to interfere with heavy chain association. A proline was added to the C-terminus to inhibit processing of the antibody by carboxypeptidases. For rapid site-directed mutagenesis of the $IgG_1$ heavy chain gene the cloning restriction sites and the 18 base pair chelating peptide sequence were engineered into the primers of three separate polymerase chain reactions.

Construction of the complete expression vector involved generating four DNA fragments from the expression vector $pNCEMG_1$ (C. B. Beidler, et al., (1988) J. Immunology 141:4053). Details of the construction of plasmid pNCEMGI may be found in European Patent Application 89302312.7, Publication Number 0 332 424, published September 13, 1989, the entire teaching of which is hereby incorporated by reference.

A first 9.0 kilobase Nru1-Sal1 fragment (Fragment-1) contained the neomycin, beta-lactamase and heavy chain variable region gene sequences.

A second 1.8 kilobase fragment (Fragment-2) was generated by PCR-amplification of the region of $pNCEMG_1$ containing the constant region gene using primers P1 and P2. Primer P1 contained 21 base pairs of sense strand sequence 5' to the Sal1 site. Primer P2 contained 30 base pairs of the antisense strand and the sequence coding for His-Trp-His-His-His-Pro, a stop codon and an XmaIII site at its 5' end. Once amplified, this fragment was cloned into pBR322 for confirmation of the desired nucleotide sequence.

A third 324 base pair fragment (Fragment-3) was generated using primers P3 and P4. Primer P3 contained at its 5' end the chelating peptide sequence, a stop codon, an XmaIII site and 30 base pairs of sense strand sequence. Primer P4 contained 30 base pairs of anti-sense strand sequence and the restriction site sequences for Nru1 at its 5' end. Fragment 3 was included because it contained the polyadenylation sequence and overlapped via its 5' end with the chelating peptide sequence of Fragment 2.

A fourth 2.1 kilobase fragment (Fragment-4) was generated by overlapping or recombinant PCR (R. Higuchi, in PCR Technology; Principles and Applications for DNA Amplification, H.A.. Erlich, ed. Stockton Press, New York, NY (1989)) by combining fragment 2 and fragment 3 and PCR amplifying with primers P1 and P4. Fragment 4 was subcloned into a TA cloning vector in substantial accordance with the teaching of the manufacturer. The TA cloning system is commercially available from the Invitrogen Corporation, San Diego, CA. The TA cloning vector contains a single 3' deoxythymidylate overhang and takes-advantage of the terminal transferase activity of thermophilic DNA polymerase which adds a single 3' deoxyadenylate residue to each end of the amplified fragment.

Fragment-4 was isolated from the TA vector by a Sal1/Nru1 restriction enzyme digest and ligated together with Fragment-1 to generate the 11.2 kilobase vector $pNCEMG_1$-CP (CP indicates the presence of the chelating peptide as part of the C-terminus of the human $IgG_1$).

The $pNCEMG_1$-CP heavy chain expression vector was transfected under standard electroporation conditions for mammalian cell gene expression (F. Tonegusso, A., et al., (1986) Mol. Cell Biol. 6:703; G. Chu, et al, (1987) Nucl. Acids Res. 15:1311; G. Andreason, et al., (1988) Biotechniques 6:650) with modifications to obtain the highest secreting clone in substantial accordance with the teaching of T. P. Condon, et al., (1991) Focus 13:33).

The clone indicating the highest expression of intact antibody-CP (hereinafter denoted CHEL-13) was selected. This clone expressed the complimentary kappa light chain in excess of the intact antibody, i.e., 30 $\mu g/10^6$ cells of kappa light chain and 2.7 $\mu g/10^6$ cells of intact antibody. The unmodified $pNCEMG_1$ vector yielded colonies secreting five-fold higher levels of intact $IgG_1$ than CHEL-13 (Beidler, C.B. et al., (1988) J. Immunol 141:4053). This difference in expression (as measured by antibody secretion) is not unusual. Lower expression of a single chain bacterial-expressed Fv containing a His-Trp-His-Trp-His-Trp-His C-terminal peptide has been reported (A. Skerra, et al., (1991) Biotechnology 9:273).

CHEL-13 cultures grown in a serum-free medium formulation in substantial accordance with the teaching of Ill, C. R., et al. (1988) Cellular and Developmental Biology 24:413. Supernatant from terminal CHEL-13 cultures was concentrated and dialyzed into buffer prior to purification of the IgG$_1$-CP by CP-IMAC. A nickel-loaded IDA HPLC column was loaded with concentrated supernatant, washed and eluted to yield a 4-peak chromatogram shown in Figure 7. Fractions from the peaks were analyzed by SDS-PAGE stained with Comassie blue. In the 12% SDS-PAGE gel (R. A. Jue, et al., (1985) Biochemistry 24:162) of Figure 8, lanes 2 and 3 show 40 µg of the starting CHEL-13 concentrate (lane 2) and 40 µg of the column flow through (lane 3). The arrow in lane 4 at the 160 kd position shows the presence of intact IgG$_1$-CP in lane 2 and its absence in the flow through fraction (lane 3). The presence of high levels of free light chain (25 kd) and light chain dimer (50 kd) are easily observed in both of these lanes. Lanes 4 and 5 (Figure 8) show fractions #13 and #20 (Figure 7 chromatogram) from the pH 5.8 and 4.25 elution steps, respectively. In lane 4 both free light chain and light chain dimer can be observed while lane 5 shows predominantly light chain dimer. Lanes 6 through 9 show fractions 31 through 34 of the glycine gradient elution in non-reduced form while lane 10 and 11 show fractions 33 and 34 respectively, in reduced form. The intact H$_2$L$_2$ and minor amounts of H$_2$L IgG$_1$-CP are seen in lanes 6-9. Reduction of these antibodies shows that they consist of heavy and light chains only as seen in lanes 10 and 11. Western blotting also confirmed that all gel bands of the protein eluted in the glycine gradient step as human kappa or human gamma-1 chains. After purification, the antibodies were diluted with 10mM EDTA and extensively diafiltered to remove most of the nickel bound to the chelating peptide.

Heretofore, it has not been possible to bind a protein on a AFM Substrate with energy sufficient to allow high resolution visualization of the protein in the AFM due to the force exerted by the AFM tip as it "drags" across the protein. Not only does the instant invention provide a method to identify the particular protein itself but also provides a method for the visualization of complexes formed with the ME.

Freshly cleaved mica was chosen as the AFM Substrate. All images are 300 by 300 nanometer areas. All images were obtained using a NanoScope II atomic.force microscope (commercially available from Digital Instruments, Santa Barbara, CA 93117) with a 12 µm Scanner and a 120 µm Si$_3$N$_4$ cantilever (K=0.6 N/m).

Figure 1 is an atomic force microscopy image of a freshly cleaved mica surface imaged in a fluid cell under PBS, pH 7.0 using a 12 micrometer scanner head and a 100 micrometer cantilever. The force is approximately $10^{-7}$ Newtons. This figure demonstrates that the freshly cleaved mica surface when imaged under these conditions is visually and atomically flat.

Figure 2 is an atomic force microscopy image of the mica surface after exposure to nickel. The nickel chloride was exposed to a freshly cleaved mica surface, rinsed, air-dried, and imaged under the same conditions as above. This photograph illustrates that the nickel impregnated mica surface is also atomically flat under these conditions. Two dimensional Fourier transform analysis of atomic scale images of nickel-coated mica showed hexagonal symmetry plus high frequency peaks not present in mica alone.

Figure 3 is an atomic force microscopy image of mica exposed to the CHEL 13 protein at a concentration of 0.5 mg/ml. CHEL 13 was exposed to a freshly cleaved mica surface and imaged under the same conditions as above. The CHEL13 protein was able to be pushed by the force of $10^{-9}$ N. The smearing of the protein by the cantilever movement demonstrates that a the CP-protein does not bind to the mica surface alone.

Figure 4 is an atomic force microscopy image of Nicoated mica exposed to the XCEM 449 antibody at a concentration of 0.5 mg/ml. The surface was prepared as above. 100 µl of 0.5 µg/ml of XCEM 449 antibody without a chelating peptide was injected into the fluid cell for 10 minutes and then flushed with PBS. Images were made under PBS using the 12 micrometer scanner head and a 100 micrometer cantilever. The force was approximately $10^{-9}$-$10^{-10}$ Newtons. The image indicates that there is no binding of the antibody to the metal derivatized surface in the absence of the additional chelating peptide incorporated into the XCEM449 antibody structure.

Figure 5 is an image of the IgG$_1$-CP preincubated on the nickel-coated mica surface at a similar concentration and in identical manner as those in above. The globular structures imaged in Figure 5 were not displaced by the cantilever. Size determination showed them to be approximately 14.0 nanometers in diameter by 3.4 nanometers tall which corresponds closely to dimensions determined by electron microscopic measurements and x-ray diffraction (H. N. Elsen, Immunology, Reprinted from Davis, Dulbecco, Elsen, Ginsberg and Wood's Microbiology, Second Edition 142 (1974) V.R. Sarma, et al., (1971) J. Biol. Chem 246:3753).

To insure that the CHEL13 is not binding to the metal derivatized mica surface specifically via the chelating peptide function of CHEL13 and not some other function, the CHEL13 chelating peptide function was saturated with nickel and exposed to a mica nickel surface. The failure of the Ni-CHEL13 to bind would indicate that the metal is indeed the medium through which binding to the mica surface is occurring. Figure 6 is an atomic force microscopy image of the nickel impregnated mica surface exposed to CHEL 13 containing bound nickel. The metal derivatized mica surface was prepared as above. The CHEL 13(0.5 µg/ml) containing the chelating peptide was pre-incubated for 30 minutes with 20 µl of nickel chloride (10 mM) to insure saturation of the CHEL13 with nickel. 100 µl of this material was injected into the fluid cell and incubated for 10 minutes with the Nickel-mica surface. The fluid cell was then flushed with PBS and imaged under PBS, using a 12 micrometer scanner head and a 100 micrometer cantilever. The force was approximately $10^{-9}$ Newtons. The failure of the CHEL13-Ni to bind to the nickel derivatized mica surface indicates that the specific binding demonstrated in Figure 5 above is indeed occurring through the metal.

The instant invention further provides a methods of using compounds of the formula I to regiospecifically anchor a molecule:

    a. to facilitate AFM Imaging

    b. for further manipulation,

    c. to create molecular wiring, and

    d. to create molecular factories.

A molecule possessing a metal chelating agent will bind to a metal ion immobilized on an AFM substrate with high specificity in a manner sufficiently stable to resist the disrupting forces encountered during interaction with a mechanical entity such as an AFM tip. In accordance with the above teaching and demonstrated in the accompanying figures, a stable CP-antibody-antigen complex was be immobilized on a mica surface and visualized by AFM.

Furthermore, the instant invention also allows for the visualization of stable protein/protein or protein/substrate interactions. These complexes may also be immobilized via a metal chelating moiety to allow visualization of not only the protein but how the protein interacts with its substrate/ligand/receptor. For example, a CP-antibody, synthesized in substantial accordance with the CP-antibody described above, could be reacted with its antigen and visualized by AFM. Such an antibody-antigen complex may be formed outside the environment of the AFM Substrate and then linked to the metal derivatized AFM Substrate surface and visualized and/or manipulated further.

Proteins other than immunoglobulins may also be incorporated in place of the antibody molecule thereby making the method of the present invention useful for the study of other protein-protein, protein-substrate, and enzyme-substrate interactions. For example, use of the compounds of the present invention would facilitate the identification and visualization of the binding pocket or active sites of proteins associated with disease states thereby facilitating the structure based design of therapeutic agents useful in the treatment of the disease states caused by these proteins. For example, the RB protein is indicated to be the major transforming agent of the retinoblastoma virus. Immobilization and visualization of the RB protein via the method of the present invention would allow the visualization of the interaction of this protein with known substrates thereby indicating the location of the active site. Consequently, therapeutic agents may be designed based on this information which would prevent the RB protein from achieving its transforming potential. Similarly, the HIV protease is known to be the essential agent for the processing of the inactive HIV polyprotein into its active subunits. Visualization of the HIV protease in accordance with the method of the present invention facilitates the structure based design of inhibitors of this enzyme in a method similar to that outline above. Such inhibitors would be useful in the treatment of AIDS.

The positioning and orientation capabilities will be useful in molecular manipulations necessary for realization of atomic environment. It is known in the art that one may use a AFM tip to move molecules about on an AFM Substrate. For example, Hansma, et al. [(1992) Science 256:1180-1184] demonstrate the ability to dissect and excise a fraction of a plasmid using the tip of a scanning probe microscope. The ability to anchor molecules firmly to the surface sufficient so that they may be moved about without disruption, by proper choice of the chelating agent, metal ion, and AFM Substrate, and scanning tip force allows one to move proteins about on the surface without dislodging them from the surface.

The ability to manipulate proteins (or other molecules) into spatial proximity with another will enable one to use the AFM tip to create "molecular wires". A molecular wire refers to an assemblage of electrically conductive ME's which are arranged on an microscopic level. For example, it is well known that many proteins are involved in the passage of electrons in biological systems. A "molecular wire" may be synthesized from a sequence of such electrically conductive biomolecules spatially oriented using a scanning probe microscope tip on an AFM Substrate. These "molecular wires" may be used in the development of more compact and efficient electrical devices such as semi-conductors and computer "chips".

A second application of the ability to orient molecules by this method enables the creation microscopic "production lines" and to create novel biosynthetic pathways. For example, the enzymes involved in many biochemical pathways are well known in the art. Their amino acid and DNA coding sequences are similarly well known. By manipulating the spatial orientation of these enzymes involved in biosynthesis of a desirable chemical entity (e.g., an antibiotic) so as to place them in close proximity with the naturally occurring predecessor and following enzymes in the pathway, a microscopic production line is created. Furthermore, since the proteins are anchored in position through the metal chelating moiety, they do not depend on the random diffusion of molecules to place one enzyme in proper proximity to the predecessor or following enzyme in the biosynthetic path. Furthermore, by substituting one or more enzymes in the pathway for an enzyme having different activity, one would be able to create biosynthetic or biodegredative pathways not found in nature.

These novel biosynthetic pathways could also be used in diagnostic systems to amplify the sensitivity of clinical or chemical testing systems. For example, the biosynthetic pathway may be arranged so as to produce a colored dye (for instance) when a certain rare molecule (indicative of a disease state) was exposed to the surface. Due to the natural catalytic activity of enzymes, the weak signal could be amplified in this manner to create simple test systems of greater accuracy and sensitivity than are currently available.

## EXAMPLES

The following Examples are merely illustrative of the practice of the invention and should not be considered to limit the scope of the invention in any way.

The construction of the 17.6 kilobase chimeric expression vector $pNCEMG_1$ has been described (C. B. Beidler, et al., (1988) J. Immunol 141:4053). The vector contains the murine variable region of anti-CEA murine heavy chain gene and the constant region from a human $IgG_1$ gene. The neomycin resistance and beta-lactamase genes are for mammalian and bacterial drug selection, respectively.

## EXAMPLE 1 VECTOR CONSTRUCTION GENERALLY

Murine hybridoma cells, designated as CEM 231.6.7, were used in the examples to derive and clone genomic DNA for variable light and heavy chain variable regions. Murine hybridoma CEM 231.6.7 was deposited on January 7, 1988 with the American Type Culture Collection, Rockville, Maryland, under the accession number ATCC HB 9620. The cloned genomic DNA from murine hybridoma CEM 231.6.7, and human peripheral blood lymphocytes, were used for the construction of the chimeric genes. Transfection of chimeric genes was accomplished by electroporation techniques, essentially as described by Toneguzzo, F., et al., Molecular and Cell. Biol., 6:703-706 (1986); and Chu, G. et al.., Nucleic Acid Res., 15:1311-1325 (1987), herein incorporated by reference. The host cells SP2/0-Ag14 hybridoma cells were the recipients of the chimeric genes. The SP2/0Ag14 hybridoma cells are available from the American Type Culture Collection, Rockville, MD.

## EXAMPLE 2, ISOLATION OF pNCEMγ

The construction of pNCEMγ, the anti-CEA IgG1 heavy chain expression vector is described in U.S Patent Application Number 07/272,577, filed November 17, 1988, (EPA 0 332 424) which is hereby incorporated by reference in its entirety. Isolation and CsCl purification of this plasmid was by standard DNA preparation methods as described in several laboratory manuals (e.g. Molecular Cloning, a laboratory manual. 2nd Ed. by J. Sambrook, E.F. Fritch, and T. Maniatis, Cold Spring Harbor Laboratory, Cold Spring Harbor, 1989)

## EXAMPLE 3. CONSTRUCTION OF PLASMID pNCEMγ-CP CONTAINING ANTI-CEA IgG1 HEAVY CHAIN WITH A C-TERMINAL SEQUENCE ENCODING HIS-TRP-HIS-HIS-HIS-PRO

The cloning strategy involved the generation of 4 DNA fragments (3 of which were modified) from the expression vector pNCEMγ.

## EXAMPLE 3 A. MAKING FRAGMENT 1

A 9.0 kilobase fragment (Fragment-1) containing all but the human constant region gene was isolated from an Nru1 to Sal1 restriction enzyme digest without further modification. This fragment was obtained by restriction digest of 10 μg of pNCEMγ DNA in TE buffer, with 20 units of each of the restriction enzymes Sall and Nrul, (commercially available from GIBCO-BRL,Gaithersburg, MD) or in separate consecutive reactions, in Universal Restriction Buffer from Stratagene (San Diego, CA). The digest was carried out for 2 hrs. at 37° C. After ethanol precipitation and resuspension in $H_2O$, the digested DNA was electrophoresed in a 0.5 % TBE agarose gel containing 0.5 μg/ml of ethidium bromide at 90 V for 45 min. Following visualization on a UV light box the 9.0 kb fragment was electrophoresed onto DEAE 81 paper from Schleicher and Schuell (Keene, NH). The fragment was eluted in 1.2M NaCl and recovered by ethanol precipitation. The eluted fragment, called Fragment 1, was then resuspended in 20 μl of $H_2O$.

## EXAMPLE 3 B. MAKING FRAGMENT 2

The second fragment (Fragment-2) was generated by PCR (in substantial accordance with the teaching of PCR Technology, edited by H.A. Erlich, Stockton Press, New York, 1989). Two oligonucleotide primers were synthesized on a DNA synthesizer (Model 8700) from Milligen Biosearch, (Bedford, MA)using the manufacturer's protocols. Primer 1, (#854) was designed to match a 21 base pair sequence 5' to the single Sall restriction site in pNCEMγ and has the sequence:

```
5'TTG-AGT-ACC-GTT-GTC-TGG-GTC-3'. (Seq. ID #11)
```

Primer 2 (#855) was designed to match approximately 30 bp of the antisense strand sequence within the CH3 region of the pNCEMγ gene and to contain 34 base pair sequence coding for His-Trp-His-His-His-Pro as well as a stop codon, and an XmaIII restriction sequence tail and has the sequence:

```
5'-TCG-GCC-GTC-GCA-CTC-ATG-GAT-GAT-GAT-GCC-AAT-
GTT-TAC-CCG-GAG-ACA-GGG-AGA-GGC-TCT-TCT-G-3'.
(Seq. ID #12)
```

The template (pNCEMγ), the 2 primers, the dNTP mix, and TAQ polymerase were mixed together and brought to final volume of 100μl in reaction buffer. 50μl of mineral oil were floated on top of the reaction and the tube was put into a DNA thermocycler from Perkin Elmer-Cetus (Emeryville, CA), set to cycle at 94°C for 1 minute, 60°C for 1 minute and 72°C for 2 min for 25 cycles. Following this PCR reaction, 5 μl of the reaction mixture was analyzed on a 0.75 % agarose gel in TBE buffer. The desired approximately 1.8 kb fragment was observed via a UV transilluminator. This fragment herein called Fragment 2 was isolated and purified as described above for Fragment 1 and was cloned into a holding vector, pBR322, (ATCC Designation 31344) and sequenced (as described in example 5) to confirm the correct nucleotide sequence of this 1.8 Kb fragment.

## EXAMPLE 3 C. MAKING FRAGMENT 3

The third fragment was generated by PCR as follows. Primer 1 (#977) matched a 13 base pair region of pNCEMγ and also provided 14 base pairs of the chelating peptide sequence, a stop codon, and a XmaIII site as a 5' tail. It has the sequence:

```
5'-GGC-ATC-ATC-ATC-CGT-GAG-TGC-GAC-GGC-CGA-3'. (Seq. ID#13)
```

Primer 2 (#978) included approx. 30bp of the antisense the strand 3' of the polyadenylation signal and an NruI restriction site. It has the sequence:

```
5'-GGG-GGA-TCC-TCG-AGT-CGC-GAG-CCG-AGG-GCA-GCC-
CCT-GGC-TGA-3'. (Seq. ID #14)
```

The PCR reagents and conditions were identical to those described above for Fragment 2. A 324 bp fragment resulted from this PCR step and was called Fragment 3.

## EXAMPLE 3 D. MAKING FRAGMENT 4

Fragment 4 was obtained by using Fragment 2 and Fragment 3 as templates for a PCR step. In this step, Primer 1 (#854) matched the 5' sequence of Fragment 2 and Primer 2 (#978) the 3' sequence of Fragment 3. The overlapping PCR reaction was carried out generating a fragment of size equal to the sum of Fragment 2 and Fragment 3 (i.e. 2134 bp). The PCR conditions were as described above. The 2134 bp Fragment 4 was cloned first into a TA holding vector and then reisolated as a 2134 bp Sall to NruI fragment which was then ligated together with Fragment 1, to form the final expression vector pNCEMγ-CP.

## EXAMPLE 4. CONSTRUCTION OF PLASMID PGCEMK(SRE) CONTAINING CHIMERIC LIGHT CHAIN IMMUNOGLOBULIN GENES

The eukaryotic expression vector containing the murine VL region fused to the human kappa gene was constructed using the vector pSV2gpt, available from the American Type Culture Collection, (ATCC Designation # 37145). One µg of pSV2gpt DNA was digested with the restriction enzyme EcoRI using 1 unit/µg of DNA in Reaction Buffer #3 (GIBCO-BRL, Gaithersburg, Maryland). The EcoRI ends were then made blunt by adding 10 µg of 5 mM each of the 4 deoxyribonucleotides dTTP, dGTP, dCTP and dATP, 2 units of Klenow enzyme and a 10x buffer (0.5M Tris HCl pH 7.5; 0.1 M $MgCl_2$; 10 mM dithiothreitol) in a total volume of 50 µl as described in Molecular Cloning, supra. The reaction was allowed to proceed for 30 minutes at room temperature and was followed by a ligation reaction in which phosphorylated ClaI linkers (2 µg) (New England BioLabs, Beverly, MA) were ligated to the 500 ng of EcoRI blunt-ended pSV2gpt in order to create a new ClaI site for the new vector. The ClaI linkers sequence was d(pCATCCGATG). Ligation reactions were carried out as described in Molecular Cloning, supra. Following ligation, excess linkers were removed by electrophoresis of the DNA and isolation of the linear pSV2gpt-ClaI fragment onto DEAE 81 paper. The isolated DNA was circularized by re-ligation. Competent HB101 cells were then transformed. The ampicillin resistant colonies resulting therefrom were analyzed by restriction enzyme digestion.

The resulting vector, pSV2gpt-ClaI, was then digested with ClaI and BamHI restriction enzymes (1 unit/µg of DNA). The chimeric vector pHKCE-10 was also digested with these two enzymes. The 4.5 kb pSV2gpt ClaI -BamHI fragment and the 9 kb ClaI -BamHI pHKCE-10 fragment were isolated on DEAE 81 paper. A standard ligation reaction was carried out using 375 ng of the 9 kb fragment insert DNA and 200 ng of the 4.5 kb vector DNA. Following transformation of HB101, a recombinant plasmid, designated pGCEMK, was identified by restriction mapping of the plasmid DNA. One further modification to this plasmid was the addition of a putative non-tissue specific enhancer comprising an approximate 80 base pair region of the human c-fos gene (Treisman, R., (1985) Cell 42, 889-902; Chen, et al. (1987) Nature 218:820-823. This 80 bp region was first synthesized and cloned into pUC19 by including AatII and HindIII restriction ends. The enhancer element was removed from pUC19 and the 3' overhang ends of these restriction sites were filled in as follows: 20 µg of AatII/HindIII digested pUC19-SRE vector were mixed with 3 µl of 10X T4 polymerase buffer (700 mM Tris, pH 7.4, 100 mM $MgCl_2$; 50 mM DTT), 3 µl of dNTP mix (0.5 mM each of dATP, dTTP, dCTP, and dGTP pH 7.0), 3 µl of water and 1 µg (5 units) of T4 DNA polymerase (commercially available from GIBCO-BRL, Gaithersburg, MD). The mixture was incubated at 37°C for 15 minutes then heated to 70°C for 10 minutes. After a phenol/chloroform extraction and ethanol precipitation, ClaI linkers were added to the ends of the 102 bp element as described above. Two copies of the enhancer element were ligated into the single ClaI site of pGCEMK as described above. This plasmid is the kappa light chain expression vector used to prepare the CEM chimeric light chain producing cell line called SRE 3.9 (ATCC No.CRL 11088, Deposited July 24,1992).

## EXAMPLE 5. DNA SEQUENCING OF VECTORS AND CLONED GENES

Sequencing of the cloned heavy chain gene pNCEMγ-CP was accomplished by standard procedures for double stranded templates using the protocols provided by the sequencing kit Sequenase® (commercially available from U.S. Biochemicals, Cleveland, Ohio), and the Bluescript®/DNA Sequencing System, (commercially available from Stratagene, Inc., La Jolla, CA) as adapted to the Genesis 2000® automated sequencer from DuPont (Wilmington, DE). From the DNA sequences obtained for the cloned heavy region gene, the amino acid sequence of the polypeptide encoded was deduced by a computer software program, MAPSEQ®, (commercially available from DNAStar, Madison, WI).

## EXAMPLE 6. TRANSFECTION OF SP2/0 CELLS GENE WITH THE CHIMERIC CONSTRUCT pGCEMK(SRE)

The light chain immunoglobulin encoding plasmid used for transfection was pGCEMK(SRE) which is described in the Example 4 above. The pGCEMK(SRE) plasmid, containing the murine variable light (Vk) gene fused to the human kappa gene, was first transfected into SP2/0 hybridoma cells by the electroporation technique described by Chu, et al. (Nucleic Acids Research 15:1311-1325 (1987)). The SP2/0 cells were grown in media containing 10% FBS and were maintained in log phase growth for the three days preceding electroporation. Twenty micrograms of the plasmid vector pGCEMK(SRE) was linearized using the restriction enzyme PvuI (1 u/µg) and the Reaction Buffer #7 from GIBCO-BRL, (Gaithersburg, Maryland). At the time of transfection the SP2/0 cells were collected by centrifugation in an IEC clinical centrifuge (800 rpm, 10 min, room temperature). Cells were then washed in Hanks Buffered Saline Solution from Gibco Laboratories (Grand Island, NY) containing 6 mM dextrose and resuspended at a final concentration of $1.0 \times 10^7$ cells/ml. 0.5 ml of cells were aliquotted into cuvettes at a density of $1 \times 10^7$ cells/ml and the linearized DNA was added. Electroporation was done using the Cell-Porator® from GIBCO-BRL (Gaithersburg, MD) with settings of 300μ and 350 volts. The electroporated cells were then resuspended in HH3 medium plus 10% fetal calf serum at a density of $2 \times 10^5$ /ml (in T75 flasks) for 72 hours. (37°C, 5% $CO_2$). Cells were then plated in the appropriate selection drug(s) at a density of $1 \times 10^5$ cells/ml in 24 well plates; SP2/0 cells containing pGCEMK(SRE) were plated in HMAX Media (50 ng/ml Hypoxanthine, 250 ng/ml Mycophenolic Acid and 50 μg/ml Xanthine), available from Sigma (St. Louis, Missouri). 200 μl of supernatant was collected from each well which contained HMAX resistant colonies. This supernatant was then assayed for the presence of a human kappa constant region which would indicate expression of the chimeric immunoglobulin light chain.

## EXAMPLE 7. IDENTIFICATION OF SP2/0 CELLS SECRETING CHIMERIC CEM LIGHT CHAIN

Transfected SP2/0 cells expressing the chimeric CEM kappa genes were identified by a standard enzyme-linked immunosorbent assay ("ELISA", as described by Engvall, E. and Perlmann, P., (1971) Immunochemistry 8:871-874 ) for human kappa. The purpose of this assay was to identify those cells secreting the chimeric kappa chain polypeptide coded for by pGCEMK(SRE) plasmid vector. A 5 μ/ml solution of goat anti-human kappa chain (Tago #4106, commercially available from Tago Inc., 887 Mitten Road, Burlingame, CA) in 10 mM sodium phosphate pH 7.4 was prepared. Each well of a 96 well plate was coated with 50 μl of this solution. The plates were then incubated overnight at 37°C. Plates were then rinsed thoroughly in $H_2O$, and then PBS + 0.1% Tween™ (w/v). Fifty μl of the supernatant fractions were added to each well, and incubated for 2 hours at room temperature. Plates were again rinsed as detailed above. A goat anti-human kappa chain alkaline phosphatase conjugate (Tago #2496, Tago Inc., 887 Mitten Road, Burlingame, CA) was diluted 1:1000 in the same medium as the supernatant material. 100 μl were added per well and allowed to incubate for 1 hour at room temperature. Plates were rinsed as above. The alkaline phosphatase substrate was prepared as per package instruction, one tablet per 3 ml of distilled $H_2O$ and 150μl of this substrate was added to each well and allowed to incubate 30 minutes at 37°C. The reaction was quenched with 50 μ of 300 mM EDTA and then the absorbance was read at 405 nM. Those supernatants showing the highest levels of kappa expression were subcloned and expanded for introduction of the plasmid construct pNCEMγ-CP.

## EXAMPLE 8. TRANSFECTION OF CHIMERIC KAPPA CHAIN PRODUCING CELLS WITH THE HEAVY CHAIN PLASMID CONSTRUCT pNCEMγ-CP

The heavy chain immunoglobulin encoding plasmid used for transfection of SRE 3.9 cells was pNCEMγ-CP, constructed as described in Example 3. A subcloned population of cells expressing the chimeric CEM kappa genes was electroporated with the plasmid construct. The SRE 3.9 chimeric kappa producing cells (SRE 3.9) were maintained at log phase of growth for the three days preceding the electroporation. Twenty micrograms of the plasmid DNA pNCEMG1-CP was linearized with the enzyme Pvul in Reaction Buffer #6 from GIBCO-BRL (Gaithersburg, MD). Cells were collected, washed and resuspended at a density of $1 \times 10^7$ cells/ml. The DNA was added and the mixture was electroporated as described in Example 6 above. Cells were plated at $2.5 \times 10^5$ cells/ml in HH4 media plus 10% fetal calf serum plus HMAX and grown for 72 hours at 37°C, 5% $CO_2$. Cells were then plated at $5 \times 10^4$/ml in 24 well plates in medium containing HMAX and G418 antibiotic (Geneticin) from GIBCO-BRL (Gaithersburg, MD) at an active concentration of 500 μg/ml. Selection was maintained for 14 days at which time those wells with HMAX/G418 resistant colonies were identified for further analysis.

## EXAMPLE 9. ELISA OF ASSEMBLED IgG1-CP ANTIBODY EXPRESSION.

Detection of assembled antibodies was carried out by coating the microtiter plate wells with goat anti-human IgG heavy chain antibody reagent (Tago #3100, Tago Inc., 887 Mitten Road, Burlingame, CA) at 5 μ/ml in 10 mM phosphate pH 7 to 8. Plates were dried overnight at 37°C, then washed with PBS and 0.1% Tween™-20, then $H_2O$. Fifty microliters of the cell supernatant were added to each well and incubated for 2 hours at room temperature. Plates were again rinsed as detailed above. A goat anti-human kappa chain alkaline phosphatase conjugate (Tago #2496 Tago Inc., 887 Mitten Road, Burlingame, CA) was diluted 1:1000 in the same medium as the supernatant material. 100 μ were added per well and allowed to incubate for 1 hour at room temperature. Plates were rinsed as above. The alkaline phosphatase substrate was prepared as per package instruction, one tablet per 3 ml of distilled $H_2O$ and 150 μl of this substrate was added to each well and allowed to incubate 30 minutes at 37°C. Purified protein from a transfectoma XCEM 449.08 was used as a positive control.

## EXAMPLE 9. QUANTITATION OF ASSEMBLED CHIMERIC ANTIBODIES.

The quantitation of intact antibody was accomplished essentially as described in the assembled Ig ELISA. Standard curves were generated by plating serial dilutions of a known concentration of the chimeric CEM antibody in a range of 5 to 180 ng/ml. The optical densities were read at 405 nm and each sample was quantitated against the appropriate standard curve.

## EXAMPLE 10. PURIFICATION OF CHEL-13 ANTIBODY

300 ml of culture supernatant from terminal cultures of CHEL-13 secreting cells grown in serum free or 1% fetal calf serum containing media was dialyzed against 10 l of Buffer A (1OOmM $NaH_2PO_4$ (pH 7.4); 1OOmM NaCl) at 4°C for 18 h. Following dialysis the media was centrifuged at 3000 x g for 10 min. The clarified media was decanted. A 4.6 x 50 mm iminodiacetate column from Perspective Biosystems (Cambridge, MA) was loaded with $Ni^{2+}$ in substantial accordance with the manufacturer's instructions. The $Ni^{2+}$ loaded column was equilibrated with Buffer A. The clarified media was loaded onto the column using an HPLC pump (model 510) from Waters (South San Francisco, CA) at a flow rate of 10 ml/min. After the media was loaded, the column was washed with 50 ml of Buffer A. The column was transferred to a Model 1090 HPLC from Hewlett Packard (Palo Alto, CA). The column was then washed with $\geqq$15 ml of Buffer A at a flow rate of 3 ml/min. The column was washed successively with 15 ml 50% Buffer A; 50% Buffer B (100 mM $NaH_2PO_4$ (pH 4.25): 100 mM NaCl) then with $\geqq$30 ml Buffer B. Following these washes the column was re-equilibrated with 6 ml Buffer A. Bound CHEL-13 was eluted with a linear gradient from Buffer A to Buffer C (1M glycine (pH 8.7); 100 mM NaCl) developed over 10 min. Fractions were collected and analyzed for antibody content by SDS-PAGE. The antibody containing fractions were dialyzed against a 800 fold excess of PBS (10mM $Na_2HPO_4$ (pH7.0); 150mM NaCl) at 4°C for 18 hours. The pooled antibody was concentrated using an Amicon® concentrating cell with a YM 10 membrane from W.R. Grace and Co. (Danvers, MA). Contaminating $Ni^{2+}$ was removed as follows: (1 ) the antibody solution was diluted 10-fold with 10 mM EDTA in PBS; (2) the antibody was reconcentrated to its original volume; (3) the antibody was then diluted 10-fold with EDTA-free PBS then concentrated to 1/2 its original volume; (4) the antibody was diluted 20-fold with EDTA-free PBS and reconcentrated; (5) step (4) was repeated until the EDTA had been diluted by at least a factor of 80,000. The final concentration of antibody was estimated by absorbance at 280 nm (assuming $A^{0.1\%}$(1 cm) = 1.4) and found to be 450 $\mu$/ml.

## EXAMPLE 11. PREPARATION OF A SUITABLE SURFACE TO CONTAIN METAL IONS

5 $\mu$l of 1 mM nickel chloride was spotted on a freshly cleaved mica surface, allowed to sit for 1 minute, and rinsed with Milli-Q® water. This was air-dried at room temperature for 20 minutes, and imaged using a NanoScope II atomic force microscope (commercially available from Digital Instruments, Santa Barbara, CA 93117) with a 12 $\mu$ Scanner and a 120 $\mu$m $Si_3N_4$ cantilever (K=0.6 N/m) under PBS (pH 7.0). Figure 1 is an atomic force microscopy image of a freshly cleaved mica surface imaged under PBS, pH 7 using a 12 micrometer scanner head and a 100 micrometer cantilever. The force is approximately $10^{-9}$ Newtons. This figure demonstrates that the freshly cleaved mica surface when imaged under these conditions is visually and atomically flat. Figure 2 is an atomic force microscopy image of the mica surface after exposure to nickel. The nickel chloride was exposed to a freshly cleaved mica surface, rinsed, air-dried, and imaged under the same conditions as above. This photograph illustrates that the nickel impregnated mica surface is also atomically flat under these conditions. Two dimensional Fourier transform analysis of atomic scale images of nickel-coated mica showed hexagonal symmetry plus high frequency peaks not present in mica alone.

## EXAMPLE 12. BINDING ANTIBODY TO NICKEL-MICA

100 $\mu$l of 0.5 $\mu$g/ml CHEC13-$IgG_1$-CP was injected into the fluid cell on a freshly cleaved mica surface, and was allowed to be in contact with the mica surface for 10 minutes. The fluid cell was then flushed with PBS, and imaged under PBS (pH 7). The force was approximately $10^{-9}$ Newtons. Smearing of the protein by the cantilever movement indicates that the protein is not binding to the mica surface alone. See Figure 3.

XCEM 449, an $IgG_1$ antibody not containing the metal binding peptide, was then tested on a nickel-mica surface. 5$\mu$l of 1 mM nickel chloride was spotted on a freshly cleaved mica surface, allowed to sit for 1 minute, and rinsed with Milli-Q® water. This was air-dried at room temperature for 20 minutes, and attached to a fluid cell. 100 $\mu$l of 0.5 $\mu$g/ml XCEM 449 was injected into the fluid cell for 10 minutes, then flushed with PBS. Smearing of the protein by the cantilever, which indicates that the protein is nonspecifically bound to the mica, is illustrated in Figure 4.

Mica-Nickel with CHEL13 was then run in the following manner. Five μl of 1 mM nickel chloride was spotted on a freshly cleaved mica surface, allowed to sit for 1 minute, and rinsed with Milli-Q® water. This was air-dried at room temperature for 20 minutes, and attached to the fluid cell. 100 μl of 0.5 μg/ml CHEL 13 was injected into the fluid cell and allowed to be in contact with the nickel-mica surface for 10 minutes. The fluid cell was then flushed with PBS and imaged. Figure 5 shows this image. Globular structures that did not move with the cantilever indicate that the material is bound to the nickel-mica surface. Size determinations of the structures showed them to be approximately 14 nanometers by 3.4 nanometer. This is comparable to measurements done by electron microscopy which yielded 15 nanometers by 3.8 nanometers. Cantilever measurement of distance is dependent upon the rigidity of the structure measured.

To demonstrate that the metal binding (chelating) peptide is actually binding to nickel, the following experiment was performed. 5 μ of 1 mM nickel chloride was spotted on a freshly cleaved mica surface, allowed to sit for 1 minute, and rinsed with Milli-Q® water. This was air dried at room temperature for 20 minutes, and attached to the fluid cell. 180 μ of CHEL13 (0.5 μg/ml) was pre-incubated for 30 minutes with 20 μ of nickel chloride (10 mM). 100 μl of this material was injected into the fluid cell and incubated for 10 minutes with the nickel-mica surface. The fluid cell was then flushed with PBS and imaged. As shown in Figure 6, the protein was moved by cantilever, yielding smeared images. This demonstrates that the binding to the surface is indeed occurring through the interaction of the metal binding (chelating) peptide with the metal coated surface.

SEQUENCE LISTING

(1) GENERAL INFORMATION:

    (i) APPLICANT: HYBRITECH INCORPORATED
        (B) STREET:         11095 TORREYANA ROAD
            CITY:          SAN DIEGO
            STATE:        CALIFORNIA
            COUNTRY:     UNITED STATES OF AMERICA
            ZIP:          92196

    (ii) TITLE OF INVENTION: METHOD OF IMMOBILIZING AND ORIENTING
         MOLECULES BY THE USE OF METAL CHELATING MOIETIES TO
         FACILITATE ATOMIC FORCE MICROSCOPY OF PROTEINS AND
         MOLECULAR SCALE MANIPULATION

    (iii) NUMBER OF SEQUENCES: 14

    (iv) CORRESPONDENCE ADDRESS:
        (A) ADDRESSEE:     C. M. HUDSON
        (B) STREET:       ERL WOOD MANOR
        (C) CITY:         WINDLESHAM
        (D) STATE:       SURREY
        (E) COUNTRY:     UNITED KINGDOM
        (F) ZIP:         GU20 6PH

    (v) COMPUTER READABLE FORM:
        (A) MEDIUM TYPE: Floppy disk
        (B) COMPUTER: Macintosh
        (C) OPERATING SYSTEM: Macintosh 7.0
        (D) SOFTWARE: Microsoft Word 5.1

(2) INFORMATION FOR SEQ ID NO:1:

    (i) SEQUENCE CHARACTERISTICS:
        (A) LENGTH: 5 amino acids
        (B) TYPE: amino acid
        (C) STRANDEDNESS: single

```
      (D) TOPOLOGY: linear

 (ii) MOLECULE TYPE: peptide


 (xi) SEQUENCE DESCRIPTION: SEQ ID NO:1:

 His Trp His Met Tyr
 1               5

(2) INFORMATION FOR SEQ ID NO:2:

     (i) SEQUENCE CHARACTERISTICS:
          (A) LENGTH: 5 amino acids
          (B) TYPE: amino acid
          (C) STRANDEDNESS: single
          (D) TOPOLOGY: linear

     (ii) MOLECULE TYPE: peptide


     (xi) SEQUENCE DESCRIPTION: SEQ ID NO:2:

     His His His Met Tyr
     1               5

(2) INFORMATION FOR SEQ ID NO:3:

     (i) SEQUENCE CHARACTERISTICS:
          (A) LENGTH: 6 amino acids
          (B) TYPE: amino acid
          (C) STRANDEDNESS: single
          (D) TOPOLOGY: linear

     (ii) MOLECULE TYPE: peptide


     (xi) SEQUENCE DESCRIPTION: SEQ ID NO:3:

     His His His His His Tyr
     1               5

(2) INFORMATION FOR SEQ ID NO:4:

     (i) SEQUENCE CHARACTERISTICS:
          (A) LENGTH: 5 amino acids
          (B) TYPE: amino acid
          (C) STRANDEDNESS: single
          (D) TOPOLOGY: linear
```

(ii) MOLECULE TYPE: peptide

(xi) SEQUENCE DESCRIPTION: SEQ ID NO:4:

```
His Trp His Trp His
1               5
```

(2) INFORMATION FOR SEQ ID NO:5:

    (i) SEQUENCE CHARACTERISTICS:
        (A) LENGTH: 5 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:5:

```
His Trp His His His
1               5
```

(2) INFORMATION FOR SEQ ID NO:6:

    (i) SEQUENCE CHARACTERISTICS:
        (A) LENGTH: 11 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:6:

```
His His His His Tyr Met His His His His Tyr
1               5                   10
```

(2) INFORMATION FOR SEQ ID NO:7:

    (i) SEQUENCE CHARACTERISTICS:
        (A) LENGTH: 5 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:7:

```
His His His His His
1               5
```

(2) INFORMATION FOR SEQ ID NO:8:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:8:

```
His Trp His His His Pro
1               5
```

(2) INFORMATION FOR SEQ ID NO:9:

    (i) SEQUENCE CHARACTERISTICS:
        (A) LENGTH: 9 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:9:

```
His Gly His Gly Gly Gly His Gly His
1               5
```

(2) INFORMATION FOR SEQ ID NO:10:

    (i) SEQUENCE CHARACTERISTICS:
        (A) LENGTH: 12 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:10:

His Gly His Gly Gly Gly Gly Gly Gly His Gly His
1               5                    10

(2) INFORMATION FOR SEQ ID NO:11:

    (i) SEQUENCE CHARACTERISTICS:
        (A) LENGTH: 21 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:11:

TTGAGTACCG TTGTCTGGGT C                                              21

(2) INFORMATION FOR SEQ ID NO:12:

    (i) SEQUENCE CHARACTERISTICS:
        (A) LENGTH: 64 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:12:

TCGGCCGTCG CACTCATGGA TGATGATGCC AATGTTTACC CGGAGACAGG GAGAGGCTCT      60

TCTG                                                                 64

(2) INFORMATION FOR SEQ ID NO:13:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: DNA (genomic)

(xi) SEQUENCE DESCRIPTION: SEQ ID NO:13:

GGCATCATCA TCCGTGAGTG CGACGGCCGA                                    30

(2) INFORMATION FOR SEQ ID NO:14:

    (i) SEQUENCE CHARACTERISTICS:
        (A) LENGTH: 42 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:14:

GGGGGATCCT CGAGTCGCGA GCGGAGGGCA GCCCCTGGCT GA                      42

## Claims

1.   A complex of the formula:

$$ME\text{-}(spacer)_x\text{-}Chelator\text{-}(M)\text{-}AFM\ Substrate \qquad (I)$$

    wherein:
       "ME" is an Molecular Entity,
       $x = 0$ or $1$,
       "M" is a transition metal ion,
    with the proviso that each [ME-(spacer)x-Chelator-(M)-] unit bound to the AFM Substrate may be the same or different.

2.   The complex of Claim 1 wherein at least one of said MEs is selected from the group consisting of proteins, enzymes, nucleotides, Immunoreactive Proteins, carbohydrates, and lipids.

3.   The complex of Claim 2 wherein said ME is a protein.

4.   The complex of Claim 3 wherein Chelator is an organic chelating agent selected from the group consisting of bidentate, tridentate, quadridentate, tripod, and macrocyclic ligands.

5.   The complex of Claim 4 wherein said Chelator is an organic chelating agent selected from the group consisting of iminodiacetic acid, nitrilotriacetic acid, terpyridine, bipyridine, triethylenetetraamine, biethylene triamine.

6. The complex of Claim 5 wherein said Chelator is iminodiacetic acid.

7. The complex of Claim 3 wherein said Chelator is a chelating peptide.

8. The complex of Claim 7 wherein said chelating peptide is selected from chelating peptides of the formulae: His-Trp-His-Met-Tyr, His-His-His-Met-Tyr, His-Trp-His-Trp-His, His-Trp-His-His-His, His-His-His-His-Tyr- Met-His-His-His-His-Tyr, His-His-His-His-His, His-Gly-His-Gly-Gly-Gly-His-Gly-His, His-Gly-His-Gly- Gly-Gly-Gly-GlyGly-His-Gly-His, and His-Trp-His-His-His-Pro.

9. The complex of Claim 1 wherein an individual [ME-(spacer)$_x$-Chelator-(M)-] unit is positioned relative to one or more other [ME-(spacer)$_x$-Chelator-(M)-] units with the proviso that each [ME-(spacer)$_x$-Chelator- (M)-] unit may be the same or different.

10. A method of making the complex of Claim 1, said method comprising the steps of:
   a. synthesizing a complex of the formula:

   $$ME\text{-}(spacer)_x\text{-}Chelator\text{-}$$

   wherein:
       "ME" is an Molecular Entity,
       $x = 0$ or 1,
   b. reacting the [ME-(spacer)$_x$-Chelator-] complex with an AFM Substrate possessing a bound transition metal ion.

11. A method of making the complex of Claim 1 said method comprising the steps of:
   a. synthesizing a compound of the formula:

   $$ME\text{-}(spacer)_x\text{-}Chelator\text{-}(M)$$

   wherein:
       "ME" is an Molecular Entity,
       $x = 0$ or 1,
       "M" is a transition metal ion,
   b. reacting the ME-(spacer)$_x$-Chelator-(M) with an AFM Substrate substantially free of bound metal ions.

12. A method of manipulating Molecular Entities on a microscopic scale, said method comprising the steps of:
   a. synthesizing a complex of the formula I,
   b. manipulating the individual ME-(spacer)$_x$-Chelator- units with the tip of a scanning probe microscope so as to achieve a desired spatial organization of the invidual ME-(spacer)$_x$-Chelator- units relative to one another.

13. A method of using compounds of the formula I to anchor proteins to an AFM Substrate to allow AFM imaging of the protein through the use of organic or peptidyl metal chelating agents.

# FIGURE 1

# FIGURE 2

# FIGURE 3

# FIGURE 4

# FIGURE 5

# FIGURE 6

**FIGURE 7**

# FIGURE 8